(19) Europäisches Patentamt
European Patent Office
Office européen des brevets

(11) **EP 1 477 170 A1**

(12) **EUROPEAN PATENT APPLICATION**
published in accordance with Art. 158(3) EPC

(43) Date of publication:
**17.11.2004 Bulletin 2004/47**

(21) Application number: **03703357.8**

(22) Date of filing: **21.02.2003**

(51) Int Cl.[7]: **A61K 31/5575**, A61P 17/00, A61P 17/04, A61P 37/08

(86) International application number:
**PCT/JP2003/001920**

(87) International publication number:
**WO 2003/070252 (28.08.2003 Gazette 2003/35)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **22.02.2002 JP 2002046301**
**02.08.2002 JP 2002226680**

(71) Applicants:
- **TAISHO PHARMACEUTICAL CO., LTD Tokyo 170-8633 (JP)**
- **Sato, Fumie Fujisawa-shi, Kanagawa 251-0026 (JP)**

(72) Inventors:
- **SATO, Fumie Fujisawa-shi, Kanagawa 251-0026 (JP)**
- **ARAI, Iwao, c/o Taisho Pharmaceutical Co., Ltd. Tokyo 170-8633 (JP)**
- **TAKANO, Norikazu, c/o Taisho Pharma. Co. Ltd. Tokyo 170-8633 (JP)**
- **TANAMI, Tohru, c/o Taisho Pharmaceutical Co., Ltd. Tokyo 170-8633 (JP)**
- **YAGI, Makoto, c/o Taisho Pharmaceutical Co., Ltd. Tokyo 170-8633 (JP)**

(74) Representative: **HOFFMANN - EITLE Patent- und Rechtsanwälte Arabellastrasse 4 81925 München (DE)**

(54) **ANTIPRURITICS**

(57) The pharmaceutical preparation of the invention, which comprises a prostaglandin or a pharmaceutically acceptable salt thereof as an effective ingredient, has an antipruritic effect with fewer side effects. It is particularly effective in controlling the itch sensation accompanying atopic symptoms.

EP 1 477 170 A1

## Description

### TECHNICAL FIELD

This invention relates to pharmaceutical preparations for preventing or treating pruritic symptoms (the pharmaceutical preparations are hereunder sometimes referred to as antipruritics), more particularly to antipruritics effective in eliminating the itch sensation due to atopic symptoms.

### BACKGROUND ART

Recent years have seen a rapid increase in the number of patients with pruritic symptoms as from atopic dermatitis, atopic conjunctivitis and senile xerosis. These diseases are accompanied by an intense itch sensation of obscure etiology and itch-evoked scratching behavior is believed to induce inflammations in mucous membranes or skin. Therefore, eliminating an itch sensation is crucial to the elimination of those symptoms.
Pharmaceutical preparations conventionally used to treat pruritic symptoms include steroids for external application, immunosuppressives, antihistamines and antiallergics. However, the use of steroids and immunosuppressives is restricted for the side effects they may cause as a result of prolonged continued use, and no antihistamines or antiallergics have been obtained that are completely satisfactory in terms of therapeutic efficacy.
Heretofore, antipruritics have been assessed by administering histamine, serotonin and other pruritogens into the skin of animals and measuring their itch-evoked scratching behavior. However, it was recently reported that the manifestation of an itch due to pruritic symptoms as in atopic dermatitis is not simply the reaction caused by histamine, etc. that are released from mast cells (J. Dermatological Science 25, 20-28, 2001).
Therefore, it is desired to develop antipruritics that depend on a new mechanism of action for preventing and treating pruritic symptoms as in atopic dermatitis.
A prostaglandin has been reported to be a prurigenic component (J. Am. Acad. Dermatol. 47, 28-32, 2002) but its use as an antipruritic is not known.

### DISCLOSURE OF THE INVENTION

An object, therefore, of the present invention is to provide pharmaceutical preparations with fewer side effects, which depend on a new mechanism of action for preventing or treating pruritic symptoms, in particular, atopic symptoms.
With a view to attaining this object, the present inventors established the method of assessment to be described later and made an investigation adopting the method, finding that prostaglandins had an outstanding antipruritic effect with fewer side effects and were particularly effective in controlling the itch sensation accompanying atopic symptoms. The present invention has been accomplished on the basis of this finding.
Thus, according to one embodiment of the invention, there is provided a pharmaceutical preparation for preventing or treating pruritic symptoms, which comprises a prostaglandin or a pharmaceutically acceptable salt thereof as an effective ingredient.
According to another embodiment of the invention, there is provided a pharmaceutical preparation for preventing or treating atopic symptoms, which comprises a prostaglandin or a pharmaceutically acceptable salt thereof as an effective ingredient.
According to another embodiment of the invention, there is provided such a pharmaceutical preparation wherein the prostaglandin is a prostaglandin agonist.
According to another embodiment of the invention, there is provided such a pharmaceutical preparation wherein the prostaglandin agonist is a prostaglandin DP receptor agonist, a prostaglandin EP receptor agonist or a prostaglandin IP receptor agonist.
According to another embodiment of the invention, there is provided such a pharmaceutical preparation wherein the prostaglandin is a prostaglandin E.
According to another embodiment of the invention, there is provided such a pharmaceutical preparation wherein the prostaglandin comprises one or more members selected from the group consisting of BW245C, ZK110841, RS93520, 15-methyl PGD2 or an optical isomer thereof, and prostaglandin D2.
According to another embodiment of the invention, there is provided such a pharmaceutical preparation wherein the prostaglandin comprises one or more members selected from the group consisting of prostaglandin D2, BW245C, ZK110841, RS93520 and 15-methyl PGD2.
According to another embodiment of the invention, there is provided such a pharmaceutical preparation wherein the prostaglandin comprises one or more members selected from the group consisting of prostaglandin E2, enprostil, sulprostone, AH13205, GR63799, M&B28767, misoprostol, 11-deoxy-PGE1, ONO-AE-248, TEI3356, 16,16-dimethyl-PGE2, 1-hydroxy-PGE1, prostaglandin E1 and limaprost.

According to another embodiment of the invention, there is provided such a pharmaceutical preparation wherein the prostaglandin comprises one or more members selected from the group consisting of prostaglandin E1, prostaglandin E2, sulprostone, enprostil and limaprost.
According to another embodiment of the invention, there is provided such a pharmaceutical preparation wherein the prostaglandin E comprises one or more members selected from the group consisting of prostaglandin E1, prostaglandin E2 and limaprost.
According to another embodiment of the invention, there is provided such a pharmaceutical preparation wherein the prostaglandin comprises one or more members selected from the group consisting of cicaprost, beraprost, iloprost, ONO-1301, carbaprostacycline, prostaglandin I2 and clinprost.
According to another embodiment of the invention, there is provided such a pharmaceutical preparation wherein the prostaglandin comprises one or more members selected from the group consisting of prostaglandin I2, cicaprost, beraprost, iloprost and clinprost.
According to another embodiment of the invention, there is provided such a pharmaceutical preparation wherein the atopic symptoms are those in atopic dermatitis or atopic conjunctivitis.
According to another embodiment of the invention, there is provided such a pharmaceutical preparation which is an agent for external application.
According to another embodiment of the invention, there is provided a method for preventing or treating pruritic symptoms, which comprises administering to a mammal a prophylactically or therapeutically effective amount of a prostaglandin or a pharmaceutically acceptable salt thereof.
According to another embodiment of the invention, there is provided a method for preventing or treating atopic symptoms, which comprises administering to a mammal a prophylactically or therapeutically effective amount of a prostaglandin or a pharmaceutically acceptable salt thereof.
According to another embodiment of the invention, there is provided use of a prostaglandin or a pharmaceutically acceptable salt thereof in the manufacture of a pharmaceutical preparation for preventing or treating pruritic symptoms.
According to another embodiment of the invention, there is provided use of a prostaglandin or a pharmaceutically acceptable salt thereof in the manufacture of a pharmaceutical preparation for preventing or treating atopic symptoms.

## BRIEF DESCRIPTION OF DRAWINGS

Figure 1 shows dermatitis scores as observed with time over 4 weeks after drug administration.
Figure 2 shows dermatitis scores as observed at 4 weeks after drug administration.

## BEST MODE FOR CARRYING OUT THE INVENTION

The antipruritics of the invention will be described below.
The present invention is characterized by comprising a prostaglandin or a pharmaceutically acceptable salt thereof.
As used herein, the phrase "a prostaglandin or a pharmaceutically acceptable salt thereof" is intended to also encompass all isomers (geometric and optical isomers), hydrates, solvates and crystalline forms.
As used herein, the term "pharmaceutically acceptable salt" is intended to include, for example, salts with alkali metals such as sodium and potassium; salts with alkaline earth metals such as calcium and magnesium; salts with, e.g., ammonia, methylamine, dimethylamine, cyclopentylamine, benzylamine, piperidine, monoethanolamine, diethanolamine, monomethylmonoethanolamine, tromethamine, lysine, tetraalkylammonium and tris(hydroxymethyl) aminomethane; salts with mineral acids such as sulfuric acid, hydrochloric acid and phosphoric acid; and salts with organic acids such as acetic acid, oxalic acid, lactic acid, tartaric acid, fumaric acid, maleic acid, methanesulfonic acid and benzenesulfonic acid.
"Prostaglandins" used herein as effective ingredients are all based on the prostanoic acid structure and are divided into types A, B, C, D, E, F, G, H, I and J, depending on differences in the number and position of oxygen atom (s) and double bond(s) attached to the 5-membered ring. Depending on the number of double bond(s) in their side chains, they are further divided into subtypes 1, 2 and 3. Prostaglandins may be either naturally occurring or synthetic.
For example, a prostaglandin D refers to a set of prostanoic acids having a 9$\alpha$-hydroxyl group and an 11-oxo group.
For example, a prostaglandin E refers to a set of prostanoic acids having a 9-oxo group and an 11-hydroxyl group.

For example, a prostaglandin I refers to a set of bicyclo prostanoic acids having an 11-hydroxyl group and a 6,9-epoxy group.

A preferred prostaglandin is a prostaglandin agonist.

The term "prostaglandin agonist" is intended to mean a substance with agonistic action on prostaglandin receptors. Compounds merely known as prostaglandin antagonists also fall within the scope of the prostaglandin agonist of the present invention, as long as they can serve as agonists depending on their dose.

As used herein, the term "prostaglandin receptors" is intended to mean receptors specific to individual prostaglandins. Such receptors are coupled to G-protein and hence trigger the signal transduction system in which cyclic AMP and inositol 1,4,5-trisphosphate serve as second messengers.

A preferred example of the prostaglandin agonist is an agonist of prostaglandin DP receptor, EP receptor or IP receptor.

Prostaglandin DP receptor agonists can be identified to have an affinity with DP receptor, as tested by binding tests using mouse DP receptor-expressing cells, and to produce a dose-dependent enhancement of cAMP production through activation of the adenylate cyclase system (Proc Natl Acad Sci U S A. 1994 Nov 8;91(23):11192-6; the disclosure of which is incorporated herein by reference).

Prostaglandin EP receptor agonists can be divided into four subtypes of receptor agonist EP1, EP2, EP3 and EP4, depending on the type of receptor. The EP1 receptor is coupled to the $Ca^{2+}$ recruitment system, the EP2 and EP4 receptors are coupled to activation of adenylate cyclase, and the EP3 receptor is coupled to inhibition of adenylate cyclase.

Prostaglandin EP1 receptor agonists can be identified to have an affinity with EP1 receptor, as tested by binding tests using mouse EP1 receptor-expressing cells, and to cause a dose-dependent increase in intracellular [$Ca^{2+}$] levels of the same cells as a result of extracellular $Ca^{2+}$ influx (see J Biol Chem 1993 Sep 25; 268(27):20175-8 and Biochim Biophys Acta 1995 May 11;1244(1): 41-8; the disclosures of which are incorporated herein by reference).

Prostaglandin EP2 receptor agonists can be identified to have an affinity with EP2 receptor, as tested by binding tests using mouse EP2 receptor-expressing cells, and to produce a dose-dependent enhancement of cAMP production in the same cells through activation of the adenylate cyclase system (see Br J Pharmacol 1986 Jan;87(1): 45-56 and Mol Pharmacol 1994 Aug;46(2):213-20; the disclosures of which are incorporated herein by reference).

Prostaglandin EP3 receptor agonists can be assigned to subtype $\alpha$, $\beta$ or $\gamma$, depending on the type of receptor. EP3$\alpha$ receptor agonists can be identified to have an affinity with EP3$\alpha$ receptor, as tested by binding tests using mouse EP3$\alpha$ receptor-expressing cells, and to cause a dose-dependent decrease in cAMP production in the same cells through inhibited activation of the adenylate cyclase system. Alternatively, they can be identified to cause a dose-dependent increase in intracellular [$Ca^{2+}$] levels of the same cells as a result of extracellular $Ca^{2+}$ influx (see J Biol Chem 1992 Apr 5;267(10):6463-6, Biochim Biophys Acta 1993 Feb 17; 1175(3):343-50, and Biochem Biophys Res Commun 1994 Oct 14; 204(1):303-9; the disclosures of which are incorporated herein by reference).

Prostaglandin EP4 receptor agonists can be identified to have an affinity with EP4 receptor, as tested by binding tests using mouse EP4 receptor-expressing cells, and to produce a dose-dependent enhancement of cAMP production in the same cells through activation of the adenylate cyclase system (see J Biol Chem 1993 Apr 15;268 (11):7759-62 and FEBS Lett 1995 May 15;364(3):339-41; the disclosures of which are incorporated herein by reference).

Prostaglandin IP receptor agonists can be identified to have an affinity with IP receptor, as tested by binding tests using mouse IP receptor-expressing cells, and to produce a dose-dependent enhancement of cAMP production in the same cells through activation of the adenylate cyclase system. Alternatively, they can be identified to cause a dose-dependent stimulation of PI (phosphatidylinositol) metabolism in the same cells (see J Biol Chem 1994 Apr 1; 269(13):9986-92 and FEBS Lett 1994 May 9;344(1):74-8; the disclosures of which are incorporated herein by reference).

Specific examples of prostaglandin DP, EP and IP receptor agonists include prostaglandin E1, prostaglandin E2, prostaglandin E3, ecraprost, clinprost, pimilprost, limaprost, ozagrel, ibudilast, ornoprostil, alprostadil, enprostil, prostaglandin I1, prostaglandin I2, prostaglandin I3, beraprost, iloprost, BW245C, ZK110841, RS93520, sulprostone, AH13205, GR63799, M&B28767, fluprostenol, cloprostenol, prostalene, cicaprost, octimibata, misoprostol, rioprostil, epoprostenol, dinoprostone, treptostinil, treprostinol, isopropyl unoprostone, gemeprost, rosaprostol, ONO-1608, ONO-8815, ONO-4819, DE-085, ecraprost (AS-013), 11-deoxy-PGE1, AY23626, ONO-1301, ONO-AE-248, TEI3356, 13,14-dehydro-15-cyclohexyl- carbaprostacycline, prostaglandin D1, prostaglandin D2, prostaglandin D3, 15-methyl PGD2 and an optical isomer thereof (e.g., 15(S)-15-methyl PGD2), 16,16-dimethyl-PGE2, carbaprostacycline, isocarbacycline, 1-hydroxy PGE1, as well as salts (e.g., sodium, calcium, lysine and tris(hydroxymethyl)- aminomethane salts) and derivatives (e.g., carboxylic esters) thereof.

Representative DP receptor agonists include BW245C, ZK110841, RS93520, 15-methyl PGD2 and an optical isomer thereof (particularly 15(S)-15-methyl-PGD2), and prostaglandin D2.

Representative EP receptor agonists include prostaglandin E2, enprostil, sulprostone, AH13205, GR63799, M&B28767, misoprostol, 11-deoxy-PGE1, ONO-AE-248, TEI3356, 16,16-dimethyl-PGE2, 1-hydroxy-PGE1, prostaglandin E1 and limaprost.

Representative IP receptor agonists include cicaprost, beraprost, iloprost, ONO-1301, carbaprostacycline, prostaglandin I2 and clinprost.

It should be noted that the compounds represented by the abbreviations mentioned above have the following common names, respectively:

BW245C: 3-[(3R)-3-cyclohexyl-3-hydroxypropyl]-2,5-dioxo-(4S)-imidazolidineheptanoic acid;
ZK110841: 7-[(1R,2R,3R,5R)-5-chloro-2-[(1E,3S)-3-cyclohexyl-3-hydroxy-1-propenyl]-3-hydroxycyclopentyl]-(5Z)-5-heptenoic acid;
RS93520: 4-[(1R,2R,3S,6R)-2-[(3S)-3-cyclohexyl-3-hydroxy-1-propynyl]-3-hydroxybicyclo[4.2.0]octa-7-ilyden]-(4Z)-butanoic acid;
ONO-8815: L-lysine(Z)-7-[(1R,2R,3R,5R)-5-chloro-3-hydroxy-2[(E)-(S)-4-(1-ethylcyclobutyl)-4-hydroxy-1-butenyl]cyclopentyl]-5-heptenoate;
ONO-1301: [7,8-dihydro-5-[(E)-[[α-(3-pyridyl)-benzylidene]amino-oxy]ethyl]-1-naphthyl-oxy]acetic acid;
AH13205: trans-2-[4-(1-hydroxyhexyl)phenyl]-5-oxocyclopentane-heptanoic acid;
GR63799: (-)-[1(R)-[1α(z),2β(R*),3α]-7-[3-hydroxy-2-(2-hydroxy-3-phenoxypropoxy)-5-oxocyclopentyl]-4-heptenoic acid 4-(benzoylamino)phenyl ester; and
TEI3356: (16S)-15-deoxy-16-hydroxy-methyl-9(0)-methano-6(9α)-prostaglandin I1.

As long as they can relieve or eliminate an itch sensation, the antipruritics of the invention are not limited in any way but they are particularly effective against the itching evoked by atopy (i.e., evoked by IgE-mediated antigen-specific immune response). From this viewpoint, the antipruritics of the invention encompass pharmaceutical preparations for preventing or treating atopic symptoms.

In the invention, the term "pruritic symptoms" means those symptoms which involve circumscribed or generalized itching and associated inflammations on the skin and mucous membranes. Examples include scabies, urticaria, eczema, xerosis (senile xeroderma and asteatotic eczema), psoriasis, dermal pruritus, and prurigo.

In the invention, the term "atopic symptoms" means those symptoms which involve atopy-evoked, circumscribed or generalized itching and associated inflammations on the skin and mucous membranes; in other words, the term refers to atopy-evoked pruritic symptoms. Examples include atopic dermatitis and atopic conjunctivitis.

In the invention, the term "atopic dermatitis" refers to a disorder that involves itching eczema as a principal lesion which undergoes repeated exacerbation and remission; this is highly likely to develop in individuals predisposed to atopy (i.e., predisposed to IgE production).

(Assessment method)

To assess the antipruritics of the invention, an assessment method was established after examination in the following cases 1 and 2. An explanation will be given of the method.

This assessment method focuses on prolonged scratching behavior in NC/Nga mice with atopic dermatitis when compared with mice of other strains.

Namely, this assessment method is a possible approach to assess drugs having an antipruritic effect on atopic dermatitis, which is characterized by extracting and measuring spontaneous scratching behavior over a prolonged duration of time in NC/Nga mice with atopic dermatitis.

NC/Nga mice used in the assessment method are known as a pathological model for atopic dermatitis. These mice spontaneously develop destructive scratching behavior and skin lesions, exhibiting atopic dermatitis-like symptoms such as massive invasion of inflammatory cells into the lesions and elevation of blood IgE levels (H. Matsuda, et al., Int Immunol. 9(3),461-466(1997); H. Suto, et al., Int Arch Allergy Immunol. 120(suppl 1), 70-75(1999)).

There is no particular limitation on the NC/Nga mice to be used, as long as they manifest atopic dermatitis. Preferred are mice aged about 8 weeks or older, and particularly preferred are mice aged 15 to 20 weeks.

Test and control drugs may be administered, e.g., by oral, intracutaneous or intraperitoneal route or used externally, and preferably used externally.

The measurement of scratching behavior is accomplished using an apparatus for detecting an animal's repeated behavior. An example of such an apparatus for detecting repeated behavior will be illustrated in detail.

NC/Nga mice manifesting atopic dermatitis are provided with magnets in both hind paws, and then housed in a space wound with a coil in a circular shape. When the mouse makes an action, the magnets move accordingly and a current flows through the coil. The moving magnets cause a change in the current, which is detected, measured and analyzed. Such an apparatus is commercially available as an itch measuring system (product of Neuroscience).

The detected scratching actions are categorized in terms of the duration of scratching and summarized for frequency, followed by statistical analysis. The duration of scratching to be extracted is 1.0 second or longer, more preferably 1.5 seconds or longer. This is because errors are further reduced when scratching actions lasting 1.5 seconds or longer are extracted.

This assessment method allowed high-accuracy assessment of drugs having an antipruritic effect on atopic dermatitis. Namely, high-accuracy assessment for antipruritic effects of drugs was achieved by measurement of destructive scratching behavior specific to a mouse model of atopic dermatitis, as distinguished from non-scratching behavior (e.g., walking) and normal scratching behavior natural to animals, which are responsible for errors.

(Case 1)

[Spontaneous scratching counts per duration compared among ICR mice, BALB/c mice, and NC/Nga mice affected and unaffected by dermatitis]

Experimental animals used were NC/Nga male mice (14 weeks old) exhibiting atopic dermatitis-like symptoms and, as control groups, ICR mice (7 weeks old), BALB/c mice (5 weeks old) and NC/Nga mice without dermatitis (8 weeks old). All the animals had a body weight of about 30 g and were divided into groups of 6 mice each.

Magnets for scratch measurement were inserted into both hind paws of each of the above animals, and the animals were tested on the next day. The experimental animals were individually placed in a measurement cage, acclimatized for 1 hour, and then measured for their scratching behavior over 24 hours using an itch measuring system (product of Neuroscience). The recorded waveforms were processed by a scratch measuring and analyzing software package (product of Neuroscience) to score scratching behavior on the basis of paw movements for durations of 0.3-0.5 second, 0.5-1.0 second, 1.0-1.5 seconds, and 1.5 seconds or longer, each movement being counted as one scratch reflex. The data was then processed by statistical analysis to determine statistical significance between mouse strains.

(Results)

The results obtained are shown in the table below.

| Spontaneous scratching counts in ICR mice, BALB/c mice, and NC/Nga mice affected and unaffected by dermatitis | | | | |
|---|---|---|---|---|
| Duration (seconds) | ICR | BALB/c | NC/Nga (unaffected) | NC/Nga (affected) |
| 0.3-0.5 | 1570 ± 160 | 1050 ± 224 | 781 ± 81 | 1419 ± 185 |
| 0.5-1.0 | 967 ± 110 | 634 ± 110 | 500 ± 58 | 976 ± 149 |
| 1.0-1.5 | 180 ± 22 | 120 ± 29 | 96 ± 14 | 358 ± 66 * |
| ≥1.5 | 85 ± 11 | 55 ± 12 | 42 ± 8 | 600 ± 64 ** |

*: $p<0.05$,
**: $p<0.01$ (Tukey test)

The number of spontaneous scratchings was measured for each group, indicating that there was no difference in scratching behavior for durations of 0.3-0.5 second and 0.5-1.0 second, whereas prolonged scratching of 1.0 second or longer, particularly 1.5 seconds or longer, was significantly frequent in NC/Nga mice with dermatitis when compared with the other groups.

The results from the above case allowed the establishment of a method for assessing drugs having an antipruritic effect, which comprises extracting and measuring prolonged scratching behavior of 1.0 second or longer, preferably 1.5 seconds or longer, in NC/Nga mice with atopic dermatitis.

(Case 2)

[Effects of external use of tacrolimus and dexamethasone on spontaneous itch-evoked scratching behavior in NC/Nga mice]

Drugs known to have an antipruritic effect were used to examine their antipruritic effect by the above-established assessment method.

Experimental animals used were 14-week-old NC/Nga male mice, each weighing about 30 g and manifesting atopic dermatitis, and were divided into groups of 6 mice each.

To perform this test, a commercially available itch measuring system (product of Neuroscience) was used.

After magnets for scratch measurement were inserted into both hind paws of each of the above animals, the animals were individually placed in a measurement cage and measured for the number of scratchings over 24 hours (the value before drug application). Then, 200 μl each of 0.1% tacrolimus (Fujisawa Pharmaceutical Co., Ltd.) and dexamethasone (Wako Pure Chemical Industries, Ltd.) dissolved in 100% ethanol were applied onto the head and dorsal skin, followed by recording again the number of scratchings over 24 hours (the value after drug application). The control group received 100% ethanol alone. The results obtained were processed to score itch-evoked scratching behavior, assuming that each paw movement lasting 1.5 seconds or longer was counted as one scratch reflex, followed by statistical analysis to determine statistical significance between the values before and after drug application.

The results obtained are shown in the table below.

| Effects of external use of tacrolimus and dexamethasone | | |
|---|---|---|
| Test group | Before drug application | After drug application |
| Control | 392 ± 104 | 352 ± 123 |
| 0.1% Tacrolimus | 600 ± 64 | 267 ± 45 ** |
| 0.1% Dexamethasone | 567 ± 77 | 286 ± 52 ** |

**: P<0.01(paired-t test)

The results demonstrated that the suppressive effect of tacrolimus and dexamethasone on spontaneous itch-evoked scratching behavior could be measured by the above assessment method.

The above assessment method was used, in turn, for the experiments shown in the test examples below, indicating that a large number of prostaglandins allow effective suppression of spontaneously-occurring itch-evoked scratching behavior in the experiments using NC/Nga mice which spontaneously develop an atopic dermatitis-like skin disease.

Prostaglandins suppressed itch-evoked scratching behavior, against which conventional antihistamines and antiallergics were not effective. Prostaglandins are therefore believed to control itching mediated by a different mechanism of action than that of these drugs.

When applied externally, prostaglandins were also shown to have the same or a higher antipruritic effect than steroids and immunosuppressives whose prolonged continued use was limited due to their side effects. Thus, prostaglandins are advantageous over these drugs in that they cause fewer side effects during external application and are available for prolonged continued use.

The antipruritics of the invention can be administered either orally, parenterally or topically.

The prophylactically or therapeutically effective amount of prostaglandins in the antipruritics of the invention can be adjusted as appropriate for the body weight of the patient, his or her age, sex, etc. Usually, the dosage is 0.1 to 100 μg per administration and one to several administrations are tolerated per day.

The antipruritics of the present invention may also be combined with an antihistamine (e.g., diphenhydramine, carbinoxamine, chlorpheniramine, ranitidine or a salt thereof, etc.) as an additional effective ingredient.

The antipruritics of the invention can be prepared as pharmaceutical compositions employing the effective ingredient in combination with carriers, vehicles and other additives that are employed in ordinary pharmaceutical formulation procedures.

Exemplary carriers and vehicles for pharmaceutical formulation procedures include water, ethanol, lactose, microcrystalline cellulose, liquid paraffin, hydrogenated oils, beeswax, squalane, stearyl alcohol, ethylene glycol and others that are in common use.

Exemplary additives are commonly employed ingredients including disintegrants (e.g., starch), binders (hydroxypropyl cellulose and low-substituted hydroxypropyl cellulose), lubricants (e.g., talc and glycerol stearate), antioxidants, preservatives (e.g., parabens), coating agents (e.g., gelatin and hydroxypropyl cellulose), coloring agents, flavoring/odorizing agents, skin color lightening agents (e.g., sodium ellagate), surfactants (e.g., sorbitan fatty acid esters), plasticizers, humectants (e.g., glycerin, propylene glycol, polyethylene glycol and hyaluronic acid), etc.

The antipruritics of the invention can be administered in various dosage forms such as those for internal application, injections and those for external application (nasal drops and eye drops), as specifically exemplified by tablets, granules, powders, capsules, liquids, gels, plasters, ointments, creams, cataplasms and aerosols.

Dosage forms for external application are preferred for the various advantages they have, such as direct applicability to the diseased area, ease of application and a reduced possibility for the occurrence of systemic side effects.

Dosage forms "for external application" include liquids for external application, aerosols, powders for external application, ointments, creams, gels, plasters, cataplasms, etc.

The following examples and test examples are provided for the purpose of further illustrating the present invention but are in no way to be taken as limiting. Various alterations and modifications can be made by the skilled artisan on the basis of the foregoing description of the invention and are also encompassed by the invention.

## EXAMPLES

### Example 1

After the individual ingredients listed below were weighed and mixed uniformly, the resulting mixed powders were tabletted by direct compression to give tablets of 300 mg each.

| | |
|---|---|
| Limaprost | 0.6 g |
| Chlorpheniramine maleate | 100 g |
| Lactose | 1700 g |
| Microcrystalline cellulose | 500 g |
| Low-substituted hydroxypropyl cellulose L-HPC (Shin-Etsu Chemical Co., Ltd.) | 500 g |
| Talc | 49.4 g |
| Hydrogenated castor oil | 50 g |

### Example 2

The ingredients listed below were weighed and mixed uniformly, followed by addition of purified water and ethanol in the volumes indicated below to give a liquid (1000 ml).

| | |
|---|---|
| Limaprost | 0.1 g |
| Ethanol | 200 ml |
| Purified water | 800 ml |

### Example 3

The individual ingredients listed below were mixed and emulsified uniformly, followed by addition of a flavoring agent in a suitable amount to give a cream (500 g).

| | |
|---|---|
| Sulprostone | 0.5 g |
| Carbinoxamine maleate | 5 g |
| Sodium ellagate | 5 g |
| Sodium hyaluronate | 3 g |
| Methylparaben | 2 g |
| Purified water | 218.5 g |
| Liquid paraffin (#70) | 50 g |
| Squalane | 100 g |
| Cetostearyl alcohol | 60 g |
| Beeswax | 20 g |
| Glyceryl monostearate | 15 g |
| Sorbitan monolaurate | 20 g |
| Propylparaben | 1 g |

### Example 4

After the individual ingredients listed below were weighed and mixed uniformly, the resulting mixed powders were filled into #2 hard capsules in an amount of 250 mg per capsule to give a capsule formulation.

| | |
|---|---|
| Limaprost | 0.5 g |
| Diphenhydramine hydrochloride | 100 g |
| Lactose | 360 g |

(continued)

| | |
|---|---|
| Microcrystalline cellulose | 500 g |
| Talc | 39.5 g |

Example 5

The individual ingredients listed below were mixed and emulsified uniformly, followed by addition of a flavoring agent in a suitable amount to give a cream (500 g).

| | |
|---|---|
| Prostaglandin E2 | 0.5 g |
| Carbinoxamine maleate | 5 g |
| Sodium ellagate | 5 g |
| Sodium hyaluronate | 3 g |
| Methylparaben | 2 g |
| Purified water | 218.5 g |
| Liquid paraffin (#70) | 50 g |
| Squalane | 100 g |
| Cetostearyl alcohol | 60 g |
| Beeswax | 20 g |
| Glyceryl monostearate | 15 g |
| Sorbitan monolaurate | 20 g |
| Propylparaben | 1 g |

TEST EXAMPLES

Test Example 1: Effect on the spontaneous, itch-evoked scratching behavior of NC mice

Twenty-week-old NC/Nga mice each weighing about 30 g and manifesting atopic dermatitis were purchased from SLC and subjected to the following experiment. A magnet was buried in both hind paws of each mouse and by detecting its magnetism, the movement of the paws was measured with an itch measuring system (product of Neuroscience). Among the scratching actions, those lasting 1.5 seconds or longer were considered itch-evoked and their number was counted continuously. Since the itch-evoked scratching behavior had a diurnal rhythm, the diurnal rhythm of each individual animal was measured for 24 hours of the day before test and only after that, 0.1% prostaglandin E2 (PGE2; Cayman Chemical) dissolved in 100% ethanol was applied to the dorsal skin in a dose of 0.2 ml/mouse. Since there was a wide range of variation among individual animals, the experimental data was scored assuming that the initial count before drug application was set to 100%. The same test was repeated using 100% ethanol as a control and dexamethasone (a steroid; Wako Pure Chemical Industries, Ltd.) and tacrolimus (an immunosuppressive; Fujisawa Pharmaceutical Co., Ltd.) as comparative drugs. The results obtained are shown in Table 1 below.

Table 1

| Test group | Concentration (%) (dosage amount) | % Occurrence of scratching behavior |
|---|---|---|
| Solvent alone (100% ethanol) | - | 90 ± 31 |
| PGE 2 | 0.1% (0.2 ml/mouse) | 46 ± 10** |
| Dexamethasone | 0.1% (0.2 ml/mouse) | 50 ± 9** |
| Tacrolimus | 0.1% (0.2 ml/mouse) | 45 ± 8** |

**: P<0.01 (paired-t test) N = 6

External application of 0.1% prostaglandin E2 strongly reduced spontaneous itch-evoked scratching behavior to the same extent as observed in dexamethasone and tacrolimus which were known to have an antipruritic effect.

Test Example 2: Effect on the spontaneous, itch-evoked scratching behavior of NC mice

Twenty-week-old NC/Nga mice each weighing about 30 g and manifesting atopic dermatitis were purchased from SLC and subjected to the following experiment. A magnet was buried in both hind paws of each mouse and by detecting its magnetism, the movement of the paws was measured with an itch measuring system (product of Neuroscience). Among the scratching actions, those lasting 1.5 seconds or longer were considered itch-evoked and their number was counted continuously. Since the itch-evoked scratching behavior had a diurnal rhythm, the diurnal rhythm of each individual animal was measured for 24 hours of the day before test and only after that, each of the test drugs dissolved in 100% ethanol was applied to the dorsal skin in a dose of 0.2 ml/mouse. Prostaglandin E2 (PGE2; Cayman Chemical), prostaglandin D2 (PGD2; Cayman Chemical), limaprost (synthetic), sulprostone (synthetic) and beraprost (synthetic) were used as drugs according to the present invention. The subsequent 24-hr itch-evoked scratching behavior was measured and the number of itch-evoked scratchings after drug application was compared with the initial count. The experimental data was processed to calculate the percent itch suppression on the basis of the total 24-hr itch-evoked scratching counts before and after drug application (Table 2).

Percent itch suppression (%) = (itch-evoked

scratching count before drug application - itch-evoked

scratching count after drug application) × 100/itch-evoked

scratching count before drug application

For significance testing, the itch-evoked scratching counts that were obtained before and after drug application from individual animals in each group treated with a specific concentration of drug were processed by a paired t-test. Differences with a significance level of 0.5% or less were determined as "significant."

The same test was repeated using 100% ethanol as a control and, as comparative drugs, dexamethasone (a steroid; Wako Pure Chemical Industries, Ltd.), tacrolimus (FK-506, an immunosuppressive; Fujisawa Pharmaceutical Co., Ltd.) and chlorpheniramine maleate (an antihistamine; Kongo Chemical Co., Ltd.) at the concentrations shown in Table 2 below, provided that chlorpheniramine maleate was administered orally. The results obtained are shown in Table 2 below.

Table 2

| Drug | Concentration (%) | % Inhibition | Significance |
|---|---|---|---|
| EtOH | 100 | 10.2 | NS |
| PGE2 | 0.1 | 54.3 | ** |
| Limaprost | 0.01 | 79.9 | ** |
| | 0.001 | 36.2 | * |
| Sulprostone | 0.01 | 24.0 | * |
| PGD2 | 0.01 | 56.3 | * |
| Beraprost | 0.1 | 56.9 | ** |
| | 0.01 | 45.6 | * |
| Dexamethasone | 0.1 | 49.5 | ** |
| Tacrolimus (FK-506) | 0.1 | 55.5 | ** |
| Chlorpheniramine | 10 mg/kg, p.o. | 3.56 | NS |

*: P<0.05,

**: P<0.01 N = 8

External application of 0.1% prostaglandin E2 strongly reduced spontaneous itch-evoked scratching behavior in NC mice to the same extent as observed in dexamethasone and tacrolimus which were known as therapeutic agents for atopic dermatitis. Moreover, prostaglandin D2, limaprost, sulprostone and beraprost showed a 10- to 100-fold stronger antipruritic effect than these conventional therapeutic agents for atopic dermatitis. On the other hand, the antihistamine chlorpheniramine maleate caused no suppression of spontaneous itch-evoked scratching behavior.

Test Example 3: Effect on dermatitis manifesting NC mouse models

By suitably modifying a method known to the skilled artisan (Jpn. J. Pharmacol. 76, 175-183 (1998) Jun Hiroi, et al. Effects of Tacrolimus Hydrate (FK-506) Ointment on Spontaneous Dermatitis in NC/Nga Mice), the following test was conducted in order to confirm the antipruritic effect of prostaglandins.

(Method)

Animals: Four-week old SPF NC mice (male) were purchased from Japan SLC; right after their arrival, the SPF NC mice were kept together with dermatitis manifesting male NC mice (older than 20 weeks) for 2 weeks under the following conditions so as to induce itch-evoked scratching behavior. A group of mice manifesting dermatitis and another group of mice not manifesting dermatitis, each consisting of four animals, were allowed to cohabit in a sawdust cage (34 × 17 × 39 cm) and kept in an animal house set at room temperature (23 ± 3°C) and at a humidity of 55 ± 15% under illumination for 12 hours (from 7:00 am to 7:00 pm).

After 2-wk cohabitation, the purchased mice were taken out of the cage and transferred into another cage, where a group of 8 animals were further kept for 14 weeks until the test. Immediately before the application of a drug, the mice were reshuffled so that the dermatitis scores would be equal among all cages and then kept, four animals per cage.

Dermatitis score: Four factors, smoothness of fur, loss of hair, bleeding and scab formation, were rated by the following scores: 0, no symptom; 1, mild symptom; 2, moderate symptom; 3, severe symptom (minimum sum, 0; maximum sum, 12).

Drug administration: To the dorsal backs of the 20-wk NC mice that were induced to manifest dermatitis as the result of cohabitation with the spontaneous dermatitis manifesting NC mice, 100% ethanol or 0.01% limaprost or beraprost (synthetic) dissolved in 100% ethanol was applied from an Eppendorf pipette in 200-μl doses seven times a week for a period of 4 weeks.

The non-treatment group was not given any treatment. Each test group consisted of 8 animals that were observed for any dermatitic symptoms once a week for a period of 4 weeks on the basis of the above dermatitis scores.

The mean value ± standard deviation was calculated from the dermatitis scores for each group, and then statistically processed by t-test to determine statistical significance between each drug administration group and ethanol-treated group.

(Results)

Figures 1 and 2 show the observed results of dermatitis symptoms (*: $P<0.05$ in the figures).

As shown in Figures 1 and 2, the groups treated with limaprost and beraprost showed a significant suppressive effect on dermatitis scores when compared to the ethanol-treated group.

<u>INDUSTRIAL APPLICABILITY</u>

The antipruritics of the invention allow specific control of inflammation (e.g., dermatitis)-inducing itching associated with pruritic symptoms, and are particularly effective against atopic symptoms. Also, the antipruritics of the invention have an outstanding antipruritic effect without side effects as observed in conventional steroids and immunosuppressives; it is therefore safe for prolonged continued use. The antipruritics of the invention further allow control of itching, against which conventional antihistamines and antiallergics are not effective.

## Claims

1. A pharmaceutical preparation for preventing or treating pruritic symptoms, which comprises a prostaglandin or a pharmaceutically acceptable salt thereof as an effective ingredient.

2. A pharmaceutical preparation for preventing or treating atopic symptoms, which comprises a prostaglandin or a pharmaceutically acceptable salt thereof as an effective ingredient.

3. The pharmaceutical preparation according to claim 1 or 2, wherein the prostaglandin is a prostaglandin agonist.

4. The pharmaceutical preparation according to claim 3, wherein the prostaglandin agonist is a prostaglandin DP receptor agonist, a prostaglandin EP receptor agonist or a prostaglandin IP receptor agonist.

5. The pharmaceutical preparation according to claim 1 or 2, wherein the prostaglandin is a prostaglandin E.

6. The pharmaceutical preparation according to claim 1 or 2, wherein the prostaglandin comprises one or more members selected from the group consisting of BW245C, ZK110841, RS93520, 15-methyl PGD2 or an optical isomer thereof, and prostaglandin D2.

7. The pharmaceutical preparation according to claim 1 or 2, wherein the prostaglandin comprises one or more members selected from the group consisting of prostaglandin D2, BW245C, ZK110841, RS93520 and 15-methyl PGD2.

8. The pharmaceutical preparation according to claim 1 or 2, wherein the prostaglandin comprises one or more members selected from the group consisting of prostaglandin E2, enprostil, sulprostone, AH13205, GR63799, M&B28767, misoprostol, 11-deoxy-PGE1, ONO-AE-248, TEI3356, 16,16-dimethyl-PGE2, 1-hydroxy-PGE1, prostaglandin E1 and limaprost.

9. The pharmaceutical preparation according to claim 8, wherein the prostaglandin comprises one or more members selected from the group consisting of prostaglandin E1, prostaglandin E2, sulprostone, enprostil and limaprost.

10. The pharmaceutical preparation according to claim 9, wherein the prostaglandin E comprises one or more members selected from the group consisting of prostaglandin E1, prostaglandin E2 and limaprost.

11. The pharmaceutical preparation according to claim 1 or 2, wherein the prostaglandin comprises one or more members selected from the group consisting of cicaprost, beraprost, iloprost, ONO-1301, carbaprostacycline, prostaglandin I2 and clinprost.

12. The pharmaceutical preparation according to claim 11, wherein the prostaglandin comprises one or more members selected from the group consisting of prostaglandin I2, cicaprost, beraprost, iloprost and clinprost.

13. The pharmaceutical preparation according to any one of claims 2 to 12, wherein the atopic symptoms are those in atopic dermatitis or atopic conjunctivitis.

14. The pharmaceutical preparation according to any one of claims 1 to 13, which is an agent for external application.

15. A method for preventing or treating pruritic symptoms, which comprises administering to a mammal a prophylactically or therapeutically effective amount of a prostaglandin or a pharmaceutically acceptable salt thereof.

16. A method for preventing or treating atopic symptoms, which comprises administering to a mammal a prophylactically or therapeutically effective amount of a prostaglandin or a pharmaceutically acceptable salt thereof.

17. Use of a prostaglandin or a pharmaceutically acceptable salt thereof in the manufacture of a pharmaceutical preparation for preventing or treating pruritic symptoms.

18. Use of a prostaglandin or a pharmaceutically acceptable salt thereof in the manufacture of a pharmaceutical preparation for preventing or treating atopic symptoms.

Figure 1

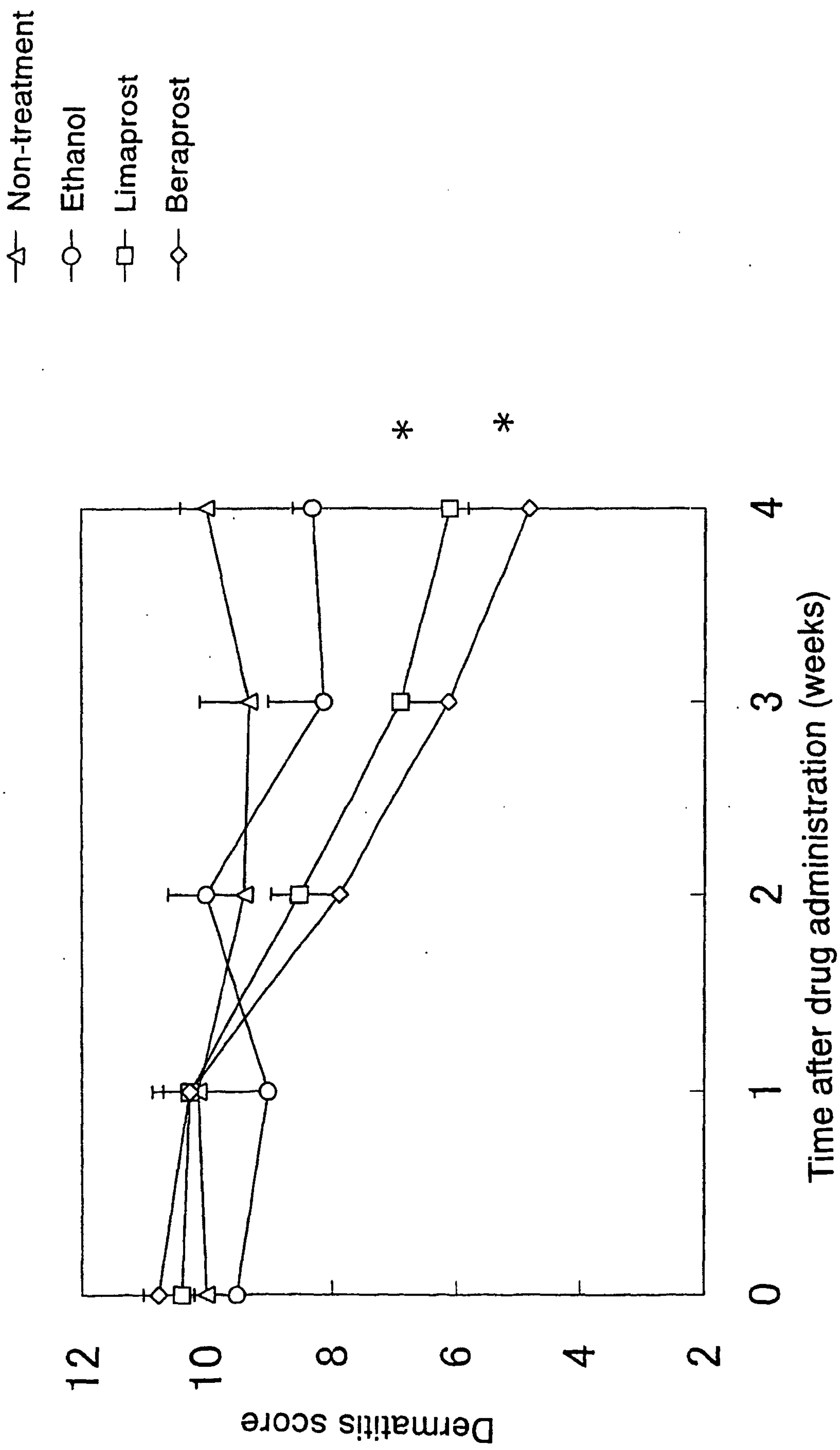

Non-treatment
Ethanol
Limaprost
Beraprost
Dermatitis score
12
10
8
6
4
2
0
1
2
3
4
Time after drug administration (weeks)
*
*

Figure 2

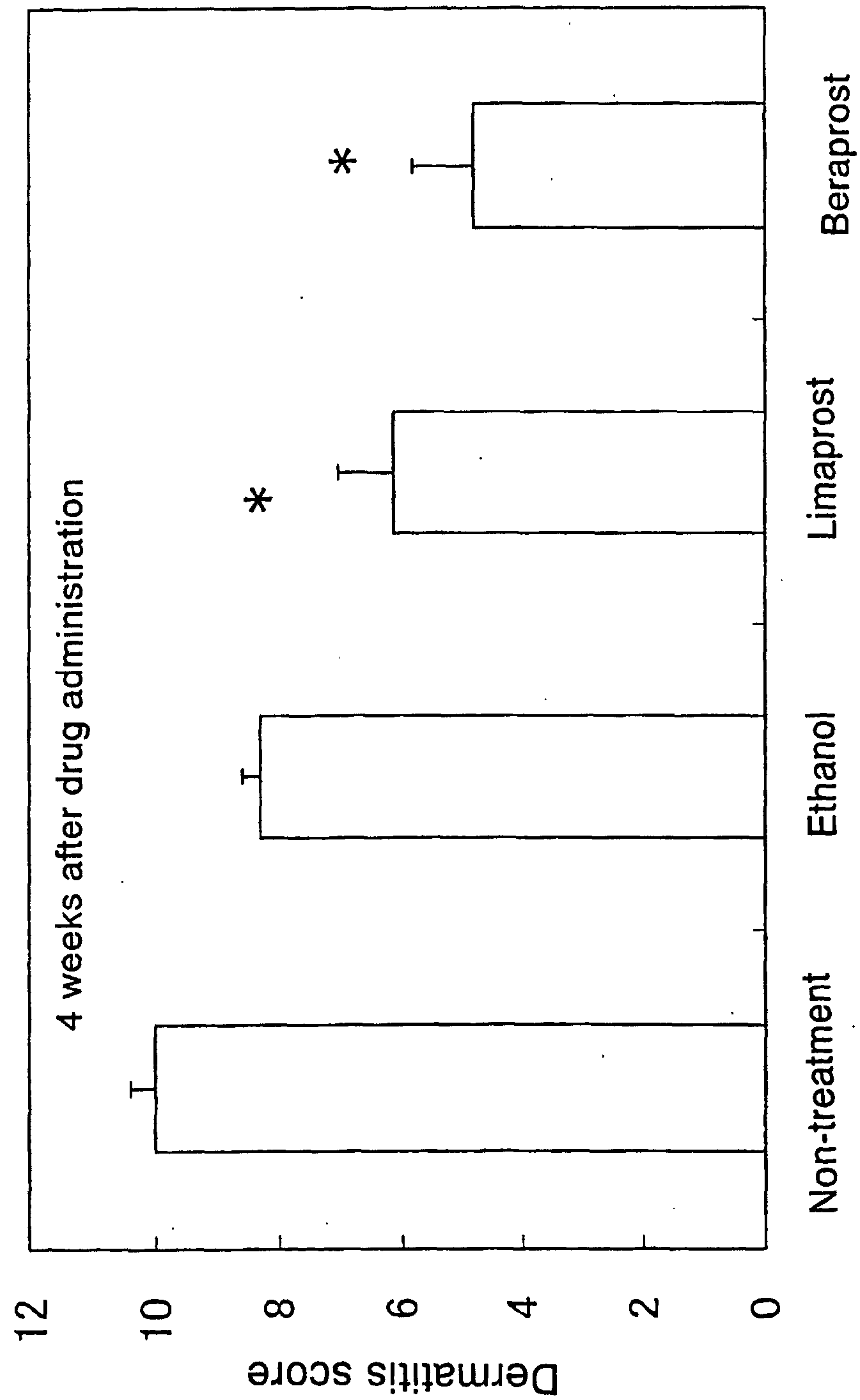
4 weeks after drug administration
Dermatitis score
0
2
4
6
8
10
12
Non-treatment
Ethanol
Limaprost
Beraprost
*
*

**INTERNATIONAL SEARCH REPORT**

International application No.
PCT/JP03/01920

A. CLASSIFICATION OF SUBJECT MATTER
Int.Cl[7] A61K31/5575, A61P17/00, 17/04, 37/08

According to International Patent Classification (IPC) or to both national classification and IPC

B. FIELDS SEARCHED

Minimum documentation searched (classification system followed by classification symbols)
Int.Cl[7] A61K31/5575, A61P17/00, 17/04, 37/08

Documentation searched other than minimum documentation to the extent that such documents are included in the fields searched

| | | | |
|---|---|---|---|
| Jitsuyo Shinan Koho | 1926-1992 | Toroku Jitsuyo Shinan Koho | 1994-1996 |
| Kokai Jitsuyo Shinan Koho | 1971-1992 | Jitsuyo Shinan Toroku Koho | 1996-2003 |

Electronic data base consulted during the international search (name of data base and, where practicable, search terms used)
CA(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| A | WO 98/27971 A1 (SLA PHARMA AG.), 02 July, 1998 (02.07.98), Full text & EP 946155 A1 & JP 2001-507020 A | 1-14,17,18 |
| A | WO 99/62555 A1 (Shionogi & Co., Ltd.), 09 December, 1999 (09.12.99), Full text & EP 1084711 A1 | 1-14,17,18 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 May, 2003 (14.05.03) | 27 May, 2003 (27.05.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.
PCT/JP03/01920

**Box I Observations where certain claims were found unsearchable (Continuation of item 2 of first sheet)**

This international search report has not been established in respect of certain claims under Article 17(2)(a) for the following reasons:

1. [X] Claims Nos.: 15, 16
because they relate to subject matter not required to be searched by this Authority, namely:
Claims 15 and 16 involve methods for treatment of the human body by surgery or therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)